# EUROPÄISCHE PATENTSCHRIFT

(11) **EP 2 635 695 B1**
(45) Veröffentlichungstag und Bekanntmachung des Hinweises auf die Patenterteilung: **28.01.2015**
(21) Anmeldenummer: 11811341.4
(22) Anmeldetag: 20.12.2011
(51) Int. Cl.: C12Q 1/02, G01N 15/14, G01N 33/50

(54) **Verfahren zum Nachweis einer Plasmodieninfektion.**
Method for detecting a plasmodium infection.
Procédé de détection d'une infection plasmodiale.

(30) Priorität: 23.12.2010 DE 102010064131; 25.01.2011 DE 102011003101
(43) Veröffentlichungstag der Anmeldung: 11.09.2013
(73) Patentinhaber: Siemens Aktiengesellschaft, 80333 München (DE)
(72) Erfinder: VAN DEN BOOGAART, Jan, NL-5711 AG Someren (NL); HAYDEN, Oliver, 91074 Herzogenaurach (DE)
(74) Vertreter: Wolff, Harry
(86) Internationale Anmeldenummer: PCT/EP2011/073428
(87) Internationale Veröffentlichungsnummer: WO 2012/084963

(56) Entgegenhaltungen:
- EP-A1- 0 545 313
- WO-A1-2005/088301
- KIM ET AL: "Subtyping lymphocytes in peripheral blood by immunoperoxidase labeling and light scatter/absorption flow cytometry.", CLINICAL CHEMISTRY, Bd. 31, Nr. 9, 1. September 1985 (1985-09-01), Seiten 1481-1486, XP55021365, ISSN: 0009-9147

## Beschreibung

Die Erfindung betrifft ein Verfahren zum Nachweis einer Plasmodieninfektion in einer Patientenblutprobe.

Plasmodieninfektionen, wie beispielsweise die durch die Erreger Plasmodium falciparum, Plasmodium vivax, Plasmodium ovale, Plasmodium malariae sowie Plasmodium knowlesi hervorgerufenen Malariaerkrankungen, sind weltweit Ursache für Hunderte von Millionen Neuerkrankungen pro Jahr. Nach Zahlen der Weltgesundheitsorganisation (WHO) wird mit jährlichen Neuerkrankungen im Bereich von 300 bis 500 Millionen Personen gerechnet.

Im Hinblick auf die zunehmende Resistenzentwicklung gegenüber den existierenden Arzneistoffen zur Behandlung von Plasmodieninfektionen besteht daher ein zunehmender Bedarf an zuverlässigen, preisgünstigen und rasch durchführbaren diagnostischen Verfahren, die zudem falsch-positive und falsch-negative Ergebnisse weitestgehend ausschließen können. Beispielsweise wären in Regionen mit einer niedrigen Prävalenz von Malariainfektionen, hochsensitive diagnostische Verfahren erforderlich, um die wenigen Erkrankten auch als solche zu erkennen.

Hingegen besteht in den Weltregionen mit hoher Prävalenz / Inzidenz, die üblicherweise auch zu den ärmeren Regionen der Welt zählen, ein hoher Bedarf an Testverfahren mit einer hohen Spezifität, um falsch-positive Ergebnisse auszuschließen (d.h. Gesunde auch als gesund zu erkennen).

In der Regel besteht zudem das Problem, dass nicht nur eine extrem hohe Anzahl an Patientenproben untersucht werden muss, sondern dass dies - wie oben angesprochen - auch rasch erfolgen muss. Denn die Ergebnisse der Diagnose sollten innerhalb weniger als 2 Stunden verfügbar sein. Falls dies nicht möglich ist, muss der Patient möglicherweise auf Grundlage einer oberflächlichen, klinischen Bewertung behandelt werden.

Die Folgen einer fehlerhaften oder falsch-negativen/falsch-positiven Malariadiagnose sind vielfältig: bei einer falsch positiven Diagnose kann der (sinnlose) Einsatz von Arzneimitteln vermeidbare Nebenwirkungen mit sich bringen, ganz abgesehen von der finanziellen Belastung für das Gesundheitssystem, sowie der Möglichkeit einer Resistenzbildung durch die Plasmodien. In einer Studie aus dem Jahre 2006 wurde geschlussfolgert, dass ein diagnostischer Test mit einer Sensitivität und Spezifität von jeweils 95 %, der nur minimale Infrastruktur benötigt, mehr als 100.000 Todesfälle und mehr als 400 Millionen unnötige Behandlungen ersparen könnte (Rafael ME, Taylor T, Magill A et al. Reducing the burden of childhood malaria in Africa: The role of improved diagnosis. Nature. 2006; 444 (suppl 1): 39 - 48)

In der Praxis ergibt sich ein Hauptproblem bei der Diagnose von Parasitenerkrankungen, beispielsweise Malaria, dadurch, dass die Labordiagnose erst dann erfolgt, wenn ein klinischer Verdacht auftritt, dass der Patient tatsächlich unter einer derartigen Infektion leidet. Dies kann insbesondere in Regionen mit einer niedrigen Prävalenz / Inzidenz dazu führen, dass erkrankte Personen nicht oder nicht rechtzeitig behandelt werden. Beispielsweise wurde in einer kanadischen Studie (Kain et al, 1998, Clinics in Infectious Diseases 27, 142 - 149) berichtet, dass bei 59 % aller mit Malaria infizierten zurückkehrenden Reisenden anfänglich nicht die richtige Diagnose gestellt wurde. Im Schnitt vergingen 7,6 Tage vor Stellung der richtigen Diagnose und vor Therapiebeginn für Plasmodium falciparum und 5,1 Tage für Plasmodium vivax. Derartige Verzögerungen können zu bedeutenden Komplikationen und zu einer erhöhten Mortalitätsrate führen (Humare et al, 1997, Canadian Medical Association Journal 156, 1165 - 1167).

Zur Diagnose von Plasmodieninfektionen stehen mehrere Verfahren zur Verfügung: das sicherste Verfahren besteht in einer mikroskopischen Blutuntersuchung, jedoch ist dieses Verfahren personal-, zeit-, und geräteaufwändig. Mit den gängigen mikroskopischen Verfahren kann das geschulte Fachpersonal die Art und das Stadium der Infektion zuverlässig bestimmen.

Daneben existieren die sogenannten raschen diagnostischen Tests (Rapid Diagnostic Test = RDT). Beispielsweise werden hier monoklonale Antikörper zum Nachweis von Parasitenantigenen verwendet. Dieser Test wird üblicherweise eingesetzt, um Plasmodium falciparum-Infektionen zu erkennen.

Ein deutlich sensitiveres Verfahren für die Malaria-Diagnostik besteht in der Polymerase-Kettenreaktion, die jedoch aufgrund des hohen Material- und Zeitaufwands für den Akutfall wenig geeignet ist.

Zur Gruppe der Rapid Diagnostic Tests (RDT)zählen mittlerweile auch automatisierte Verfahren, die sich aufgrund ihres hohen Durchsatzes für flächendeckende Nachweisverfahren hervorragend eignen. Siehe hierzu Hanscheid T, Pinto BG, Pereira I, Christino JM, Valadas E (1999) Avoiding misdiagnosis of malaria: a novel automated method allows specific diagnosis, even in the absence of clinical suspicion. Emerging Infectious diseases [1999, 5(6): 836-838].

Zur automatisierten Anwendung werden so genannte "automated cell counters" (Automatisierte Zellzählgeräte) mit zunehmendem Erfolg angewendet. Beispiele hierfür sind das Advia 2120, Sysmex XE-2100 sowie CellaVision DM96 Gerät. Diese automatisierten Geräte stellen, abgesehen von ihrer hohen Durchsatzzahl, einige Vorteile bereit, wie beispielsweise hohe Objektivität (keine Variabilität abhängig vom Beobachter), Elimination statistischer Variationen, die üblicherweise mit einer manuellen Zählung verbunden sind (Zählung hoher Zellzahlen), sowie die Bestimmung zahlreicher Parameter, die bei einer manuellen Auszählung nicht verfügbar wären und, wie angesprochen eine effizientere und kosteneffektivere Handhabung. Einige dieser Geräte können 120 bis 150 Patientenproben pro Stunde bearbeiten.

Die technischen Prinzipien der automatischen Einzelzellzählung beruhen entweder auf einer Impedanz- (Widerstands-) Messung oder auf einem optischen System (Streulicht- oder Absorptionsmessung).

Beim Impedanzverfahren erfolgt die Zellzählung sowie deren Größenbestimmung auf Grundlage des Nachweises und der Messung von Veränderungen in der elektrischen Leitfähigkeit (Wider-stand), die durch ein Teilchen verursacht werden, das sich durch eine kleine Öffnung hindurchbewegt. Teilchen, wie beispielsweise Blutzellen, sind selbst nicht leitend, werden jedoch in einem elektrisch leitenden Verdünnungsmittel suspendiert. Wenn eine derartige Suspension von Zellen durch eine Öffnung hindurchgeleitet wird, nimmt bei Durchgang einer einzelnen individuellen Zelle die Impedanz (Widerstand) des elektrischen Weges zwischen den beiden Elektroden, die sich auf jeder Seite der Öffnung befinden, vorübergehend zu.

Beispielsweise wird in WO 2005/088301 ein Verfahren zum Nachweis von Malaria und anderen Parasiteninfektionen mittels einer derartigen Impedanzmessung (siehe experimenteller Teil) sowie im Artikel "Development of an Automated Malaria Discriminant Factor Using VCS Technology", Briggs C et al. Am J Clin Pathol 2006, beschrieben.

Im Gegensatz zum Impedanzverfahren umfasst das optische Verfahren das Durchleiten eines Laserlichtstrahles durch eine verdünnte Blutprobe, die in einem kontinuierlichen Strom von dem Laserstrahl erfasst wird. Jede Zelle, die durch die Erfassungszone der Durchflusszelle hindurchtritt streut das fokussierte Licht. Das gestreute Licht wird dann durch einen Fotodetektor nachgewiesen und in einen elektrischen Impuls umgewandelt. Die hier erzeugte Anzahl von Impulsen ist direkt zur Zellanzahl proportional, die durch die Erfassungszone in einer speziellen Zeitspanne hindurchtritt.

Bei den optischen Verfahren wird die Lichtstreuung der einzelnen Zelle, die durch die Erfassungszone hindurchtritt, in verschiedenen Winkeln gemessen. Hierdurch werden Informationen über Zellstruktur, Form und Reflexionsvermögen erfasst. Diese Eigenschaften können dazu verwendet werden, verschiedene Arten von Blutzellen zu differenzieren und die abgeleiteten Parameter zur Diagnose von Abweichungen der Blutzellen von der Norm zu verwenden.

Die durch beide Messverfahren erzielten Werte werden mittels der Differentialdiagnostik zu einem aussagekräftigen Diagnoseresultat verknüpft.

Die Sensitivität und Spezifität von diagnostischen Verfahren spielt im Rahmen der Differentialdiagnostik eine tragende Rolle, dementsprechend wird fortwährend an einer Verbesserung dieser Eigenschaften gearbeitet.

Beispielsweise werden in WO 2005/088301 die Messwerte bezüglich des Zellvolumens von Lymphozyten und Monozyten erfasst und als Parameter für eine Malariaerkrankung herangezogen. Genauer gesagt wird hier die Standardabweichung des Volumens der Monozyten- und Lymphozytenpopulationen bewertet, d.h. deren Heterogenität. Es hat sich jedoch herausgestellt, dass dieser Parameter für die Diagnose einer Parasiteninfektion nicht spezifisch genug ist, da es auch bei jeder anderen Infektionskrankheit (beispielsweise Erkältungen) zu einer Volumenveränderung der Lymphozyten und Monozyten kommen kann. Darüber hinaus haben sich Impedanzmessungen von Blutproben als fehleranfällig erwiesen, z.B. können Messergebnisse (beispielsweise durch variierende Viskosität der zu testenden Suspension) verfälscht werden.

In ähnlicher Weise wird in "Development of Automated Malaria Discriminant Factor Using VCS Technology" (s.o.) beschrieben, dass die Standardabweichung des Volumens von Lymphozyten und Monozyten signifikant vom Normwert abweicht, wenn eine Malariainfektion vorliegt.

Eine beispielhafte Darstellung verschiedener Sensitivitäten und Spezifitäten unter Verwendung automatisierter Bluttestgeräte findet sich in Tabelle 3, wonach die Sensitivität teilweise nur 48,6 bzw. 52 % beträgt. Damit gelingt es mit den gängigen Testmethoden nicht im ausreichendem Maße, Verfahren mit hoher Sensitivität und Spezifität bereitzustellen, d.h. Verfahren zum Nachweis einer Plasmodieninfektion, die einen erkrankten Patienten auch als solchen erkennen, sowie andererseits einen gesunden Patienten als gesund erkennen können.

Nach neueren Untersuchungen ist die Aussagekraft der bestehenden automatisierten Testsysteme (und damit auch der angegebenen Sensitivitäten /Spezifitäten) zudem fraglich und lässt Spielraum für Verbesserungen zu. Siehe hierzu das kürzlich veröffentlichte Paper "Automated haematology analysis to diagnose malaria", Malaria Journal 2010, 9:346.

Es ist daher eine Aufgabe der vorliegenden Erfindung, ein Verfahren zum Nachweis einer Plasmodieninfektion in einer Blutprobe bereit zu stellen, das Testergebnisse mit hoher Sensitivität und Spezifität ergibt. Es ist eine weitere Aufgabe der Erfindung, ein Nachweisverfahren für Malariainfektionen bereit zu stellen, das durch den Einsatz mehrerer unabhängiger Parameter eine hohe Spezifität und Sensitivität für Malaria unabhängig von anderen Infektionen oder unterschiedlichem Gesundheitszustand der Patienten erlaubt. Es ist eine noch weitere Aufgabe der vorliegenden Erfindung, ein solches Verfahren bereitzustellen, das mit Hilfe automatisierter Blutanalysegeräte durchgeführt werden kann.

Diese Aufgabe wird erfindungsgemäß durch ein Verfahren mit den in Patentanspruch 1 angegebenen Merkmalen gelöst.

Die Erfindung schafft ein Verfahren zum Nachweis einer Plasmodieninfektion in einer Patientenblutprobe, umfassend
a) Durchführen einer Differentialanalyse der polymorphkernigen neutrophilen Granulozyten in der Probe und Bestimmen der Verteilung des Zellvolumens und der Zelldichte;
b) Bestimmen der Thrombozytenanzahl in der Probe;
c) Bestimmen der Verteilung der Zelldichte der Thrombozyten in der Probe;
d) Gewinnen von Probenparametern aus den in a) bis c) durchgeführten Bestimmungen; und
e) Bewerten der Parameter gegenüber einem vorherbestimmten Kriterium, wobei bei Erfüllung des Kriteriums eine Plasmodieninfektion vorliegt, wobei das Kriterium auf Grundlage einer oder mehrerer Probenparameter etabliert wird und auf Grundlage eines Vergleichs von infizierten Blut proben mit entsprechenden werten von normalen Blutproben bestimmt wird.

Bei der erfindungsgemäß untersuchten Patientenblutprobe handelt es sich in der Regel um eine humane Blutprobe. Es ist jedoch auch möglich, Blutproben von Säugetieren zu untersuchen.

Der Begriff "Differentialanalyse" wie hierin verwendet bedeutet die Erfassung mehrerer messbarer Einzelwerte der Bestandteile der Patientenblutprobe, die letztendlich für die durchzuführende Diagnose kombiniert ausgewertet werden.

In Schritt a) des erfindungsgemäßen Verfahrens wird hierzu zunächst eine Differentialanalyse der polymorphkernigen neutrophilen Granulozyten in der Probe bezüglich der Bestimmung der Verteilung des Zellvolumens und der Zelldichte durchgeführt. Dies schließt jedoch nicht aus, dass weitere Leukozytenarten, beispielsweise eosinophile oder basophile Granulozyten ebenfalls in die Untersuchung mit eingeschlossen werden.

Eine Abweichung der Verteilung des Zellvolumens sowie der Zelldichte von der Norm weisen in der Regel auf einen pathologischen Zustand hin. Im vorliegenden Fall weisen gegenüber den üblicherweise gemessenen Normalwerten niedrigere Werte auf eine Plasmodieninfektion hin. Die mit der Plasmodieninfektion einhergehende Verringerung des Zellvolumens sowie der Zelldichte ist mit den im Zuge der Infektion auftretenden Verteidigungsmechanismen der Leukozyten zu erklären. Beispielsweise wandern neutrophile Granulozyten aus dem Blutgefäß in das Gewebe ein, sezernieren dort proteolytische Enzyme um interzelluläre Verbindungen zu lösen und phagozytieren dort Bakterien. Hierdurch kommt es zu einer Veränderung des Zellvolumens und der Zelldichte.

Es hat sich überraschenderweise herausgestellt, dass die Sensitivität und Spezifität des Nachweisverfahrens für eine Plasmodieninfektion deutlich erhöht werden kann, wenn neben den oben beschriebenen Bestimmungen (d.h. sequentiell oder gleichzeitig) sowohl die Thrombozytenanzahl in der Patientenblutprobe als auch die Verteilung der Zelldichte der Thrombozyten in der Probe bestimmt wird. Auch hier ist ein von der Norm nach unten abweichender Wert prädiktiv für das Vorliegen einer Plasmodieninfektion. Dies führt erfindungsgemäß zu einer gegenüber bestehenden Verfahren deutlich erhöhten Sensitivität und Spezifität des Nachweisverfahrens.

Aus den in den Schritten a) bis c) erzielten Messergebnissen werden Probenparameter gewonnen und gegenüber einem vorherbestimmten Kriterium bewertet, wobei bei Erfüllung des Kriteriums eine Plasmodieninfektion vorliegt.

Bei Parametern handelt es ich um abgeleitete Größen, beispielsweise steht der Begriff Zellvolumenparameter für einen mit der Zellvolumenverteilung in Beziehung stehenden Parameter wie beispielsweise das durchschnittliche Zellvolumen oder die Standardabweichung der Zellvolumenverteilung einer gegebenen Zellsubpopulation.

Der Begriff "vorherbestimmtes Kriterium" wie hierin verwendet betrifft ein Kriterium, das auf Grundlage einer oder mehrerer Probenparameter etabliert wurde, im Falle der vorliegenden Erfindung insbesondere aufgrund von Zellvolumenparametern, Zellzahlparametern und Zelldichteparametern. Das Kriterium wird auf Grundlage eines Vergleichs von infizierten Blutproben mit entsprechenden Werten von normalen Blutproben bestimmt, beispielsweise wurden für die experimentellen Untersuchungen, die der vorliegenden Erfindung zugrunde liegen ein Vergleich von 204 *P. falciparum* infizierten Blutproben mit entsprechenden Werten von 3240 normalen Blutproben durchgeführt.

Durch Kombination und Bewertung von Probenparametern polymorphkerniger neutrophiler Granulozyten (Zellvolumen und Zelldichte) und, zusätzlich, unter Heranziehung der Thrombozytenanzahl sowie der Verteilung der Zelldichte der Thrombozyten in der Probe, konnten erfindungsgemäß unerwartet hohe Werte für die Sensitivität und Spezifität des Nachweisverfahrens erzielt werden. Wie in den Beispielen dargelegt wird, gelang es hierbei, Spezifitätswerte von 99 % und Sensitivitätswerte von 98 % erreichen. Dies bedeutet, dass bei flächendeckenden, automatisierten Blutuntersuchungen die Anzahl der falsch-positiven bzw. falsch-negativen Diagnoseergebnisse in einem bisher nicht bekannten Maße reduziert werden konnte und beinahe vernachlässigt werden kann. Dies bedeutet einen großen Fortschritt in Hinblick auf eine gesicherte Plasmodieninfektionsdiagnose, insbesondere Malaria-Diagnose und wird in Ländern, die von einer hohen Prävalenz / Inzidenz von Malariainfektionen betroffen sind zu einer deutlichen Verbesserung des Gesamtgesundheitszustandes über rechtzeitige und sinnvolle medizinische Maßnahmen führen.

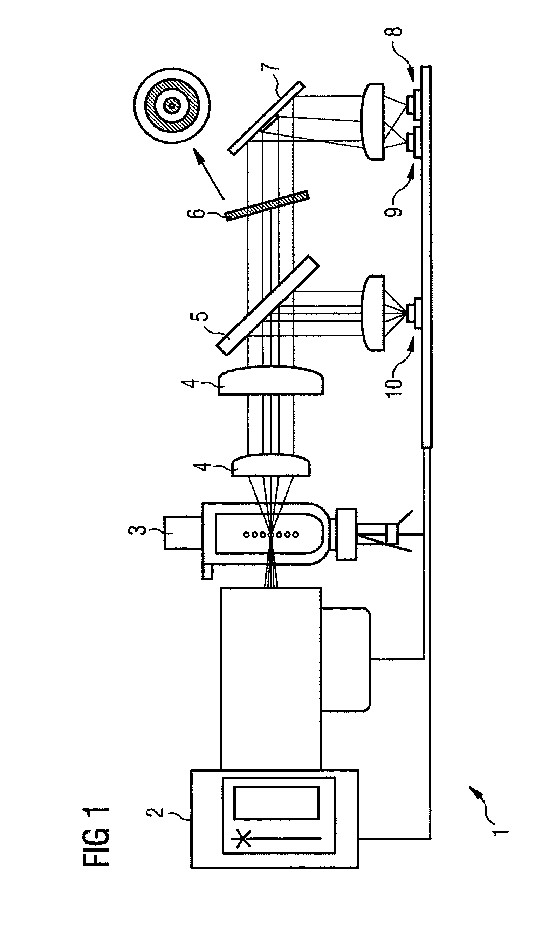

In einer Ausführungsform des erfindungsgemäßen Verfahrens erfolgt die Parameterbestimmung durch Streulichtmessung. Bei der Streulichtmessung handelt es sich, wie oben ausgeführt ("optisches Verfahren") im Gegensatz zur Impedanzmessung um ein Verfahren, bei dem Laserlicht verwendet wird, wobei die Blutproben (Zelle für Zelle) durch das Laserlicht geführt werden und die Ablenkung der Laserstrahlen durch eine geeignete Vorrichtung erfasst werden kann. Das unter Verwendung des Laserstrahls durchgeführte Verfahren ist in der beiliegenden Figur 1 erläutert. Hierbei werden die von einer individuellen Zelle gestreuten Lichtstrahlen in verschiedenen Winkelbereichen erfasst (niedriger Winkel / low angle und großer Winkel / high angle), die jeweils Hinweise auf Volumen (kleiner Winkel) sowie Dichte (großer Winkel) zulassen. Erfindungsgemäß wird als "kleiner Winkel" ein Winkel von ca. 2° bis 3° bezeichnet, als ein "großer Winkel" ein Winkel von ca. 5° bis 15° Abweichung von der Laserlichtachse. Es hat sich herausgestellt, dass die Streulichtmessung der Impedanzmessung im Hinblick auf die geringere Störanfälligkeit der Messergebnisse überlegen ist.

In einer Ausführungsform handelt es sich bei der PlasmodienInfektion um eine Infektion mit P. ovale (Malaria tertiana), P. vivax (Malaria tertiana), P. malariae (Malaria quartana) oder P. falciparum (Malaria tropica). Das erfindungsgemäße Nachweisverfahren kann für diese Plasmodieninfektionen gleichermaßen verwendet werden.

In einer Ausführungsform werden zusätzlich zu den oben genannten Verfahrensschritten zusätzlich eine Sphärisierung und Differentialanalyse der Retikulozyten und Erythrozyten in der Patientenblutprobe durch Streulichtmessung durchgeführt. Auch hier handelt es sich um eine Differentialanalyse, wobei die letztendlich zu bewertenden Parameter das Zellvolumen, die Zelldichte sowie den Hämoglobingehalt der Zellen und den Retikulozytenanteil betreffen. Bei Retikulozyten handelt es sich um junge rote Blutkörperchen, die im Gegensatz zu den Erythrozyten selbst noch RNA enthalten und dadurch differenziert werden können.

Die Sphärisierung der Erythrozyten oder Retikulozyten ist notwendig, um die Blutzellen unabhängig von ihrer ursprünglichen Form in eine bei der Streulichtmessung auswertbare Form zu überführen. Zu diesem Zweck wird die Blutprobe mit einem Reagens versetzt, das zu einer Sphärisierung der Retikulozyten und Erythrozyten führt. Beispielhafte Reagenzien sind in US 5,045,472, US 5,284,771, US 5,633,167 sowie US 6,114,173 offenbart. Beispielsweise offenbart US 5,045,472 ein Reagenzgemisch umfassend eine isotonische wässrige Lösung, ein Sphärisierungsmittel (beispielsweise ein Detergens wie Alkalimetallsalze eines Alkylsulfats) sowie ein Protein, das reversibel das Spärisierungsmittel bindet.

Durch Einbeziehung der Retikulozyten und Erythrozyten in das erfindungsgemäße Nachweisverfahren können Spezifität und Sensitivität weiter erhöht werden. Das Verhältnis von Retikulozyten zu Erythrozyten ist ein wichtiger Hinweis auf das Vorliegen einer Plasmodieninfektion im Blut. Je höher das Verhältnis der Retrikulozyten, d.h. junger, unreifer Erythrozyten im Verhältnis zu reifen Erythrozyten ist, desto höher ist die Wahrscheinlichkeit des Vorliegens einer Plasmodieninfektion.

In einer weiteren Ausführungsform wird nach einer Peroxidase-Einfärbung aller Leukozyten in der Blutprobe eine Differentialanalyse durch Streulichtmessung und Absorption durchgeführt.

Die Peroxidase-Einfärbung erfolgt üblicherweise durch zelluläre Peroxidase-Aktivität nach Umsetzung mit 4-Chlor-1-Naphtol. 4-Chlor-1-Naphtol dient hier als Substrat, das es Wasserstoffperoxyd ermöglicht, ein dunkles Präzipitat an endogenen Orten der Peroxidase-Aktivität in den Granula der Leukozyten zu bilden. Bei einer Streulichtmessung werden dann die Zellen mit einer geringen oder moderaten Peroxidase-Aktivität weniger Licht absorbieren, während Zellen mit einer hohen Peroxidase-Aktivität mehr Licht absorbieren werden.

In einer weiteren Ausführungsform werden zusätzlich nach einer Peroxidase-Einfärbung, wie vorher geschildert, die Volumenverteilung der neutrophilen Granulozyten gemessen. Auch hier gilt, dass, je größer die Volumenverteilung der neutrophilen Granulozyten ist, desto größer die Wahrscheinlichkeit für das Vorliegen einer Plasmodieninfektion in der Patientenblutprobe ist.

In einer weiteren Ausführungsform wird zusätzlich als Parameter die Standardabweichung der Volumenverteilung aller Leukozyten nach Peroxidase-Einfärbung verwendet. Je größer die Standardabweichung der Volumenverteilung aller Leukozyten ist, desto größer ist die Wahrscheinlichkeit des Vorliegens einer Plasmodieninfektion.

Bei einer Ausführungsform wird die Bestimmung in a), d.h. die Durchführung einer Differentialanalyse der polymorphkernigen neutrophilen Granulozyten in der Probe nach Lysieren wenigstens der eosinophilen und neutrophilen, nicht jedoch der basophilen Leukozyten (BASO Lysereagenz), durch ein geeignetes Lysereagenz durchgeführt. Auch hier ist das Vorliegen einer breiten Volumen- bzw. Dichteverteilung ein Indiz für das Vorliegen einer Plasmodieninfektion. Als Lysereagenz haben sich hier Gemische auf Basis von Phthalsäure und Detergenzien als vorteilhaft herausgestellt, beispielsweise kann ein derartiges Reagenz Salzsäure, Phthalsäure, ein Tensid sowie wahlweise ein Konservierungsmittel enthalten. Das Verhältnis von Phthalsäure und Salzsäure ist hierbei ungefähr 2,5:1 (basierend auf ihrer Konzentration in mmol/l. Durch dieses Reagenz werden die roten Blutkörperchen, die Thrombozyten, sowie alle Leukozyten (außer den basophilen Granulozyten)lysiert. Auch durch dieses zusätzliche Verfahren kann der Plasmodienspezifische Vorhersagewert erhöht werden.

In einer weiteren Ausführungsform wird nach einer spezifischen Lyse aller Zellen außer der basophilen Leukozyten der unspezifische Anteil aus dem Streulichtdiagramm (Klein- und Großwinkelstreuung) bestimmt wird. Je höher dieser Anteil ist, desto höher ist die Wahrscheinlichkeit, dass eine Plasmodieninfektion vorliegt.

Die im erfindungsgemäßen Nachweisverfahren durchgeführten Bestimmungen a), b) und c) sind, wie oben ausgeführt, prädiktiv für das Vorliegen einer Plasmodieninfektion. Der Begriff "niedrige" Werte im Kontext der vorliegenden Erfindung bedeutet einen niedrigeren Wert der im Einzelfall durchgeführten Bestimmung im Vergleich zu den bei normalen Patientenproben (d.h. nicht infizierten Patientenproben) erzielten Standardwerte.

Im Gegensatz hierzu bedeutet die zusätzliche Heranziehung der Parameter, die durch Peroxidase-Einfärbung sowie Bestimmung der Volumenverteilung der neutrophilen Granulozyten sowie der Standardabweichung der Volumenverteilung aller Leukozyten erzielt wird, sowie die Bestimmung des unspezifischen Anteils aus dem Streulichtdiagramm nach spezifischer Lyse aller Zellen, dass, je höher die Werte sind, desto höher die Wahrscheinlichkeit für das Vorliegen einer Plasmodieninfektion ist.

Des Weiteren werden Ausführungsformen des erfindungsgemäßen Verfahrens unter Bezugnahme auf die beigefügten Figuren detailliert beschreiben.

Es zeigen:
Figur 1 eine schematische Darstellung einer durchflusszytometischen Vorrichtung 1 zur Durchführung des erfindungsgemäßen Verfahrens.

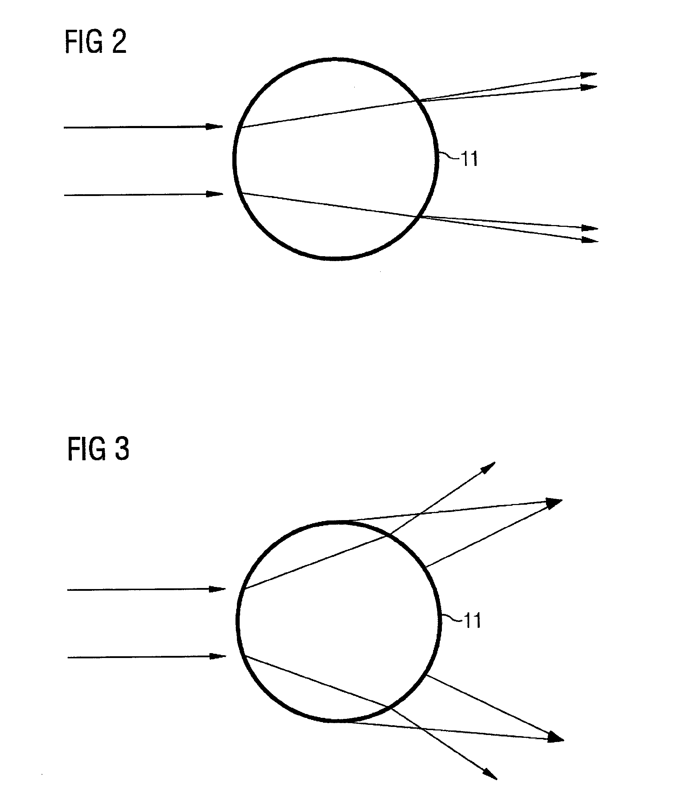

Figur 2 eine schematische Darstellung des Groß-Winkel-Streulichts anhand einer roten Blutzelle 11, das mit der Granularität und Dichte der Zelle korreliert und in einem typischen Bereich von 5° bis 15° gemessen wird. Dies entspricht der Bestimmung der Zelldichte.
Figur 3 die Bestimmung des Zellvolumens (bzw. der Zellgröße) der Zelle 11 im Klein-Winkel-Streulichtbereich (2° bis 3°).

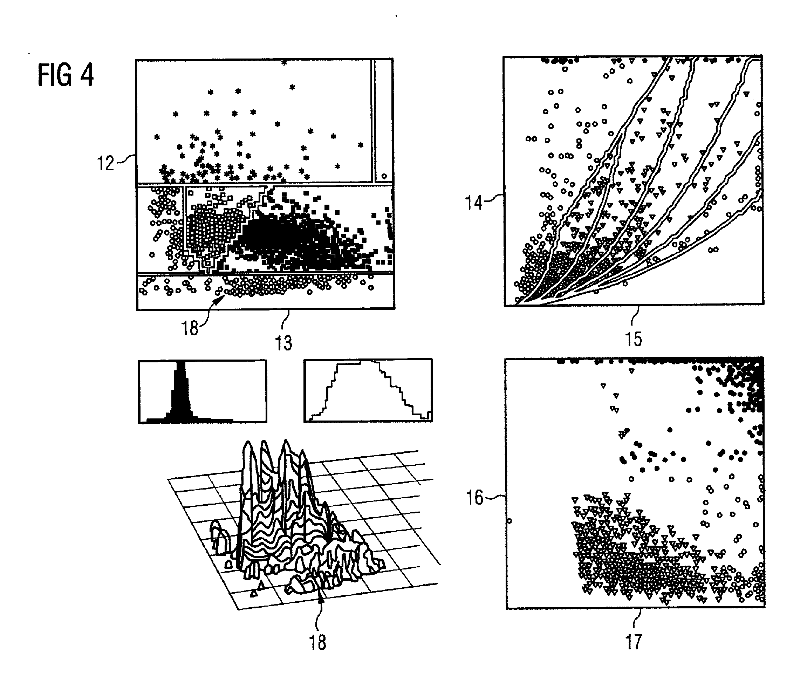

Figur 4 Streulichtdiagramme, durchgeführt mit dem Gerät ADVIA 2120i, das die beispielhafte Verschiebung des Volumens 16 und der Dichte 17 für Thrombozyten und polymorphkernige Leukozyten (PMNx) bei Malaria-infizierten Blutproben zeigt.

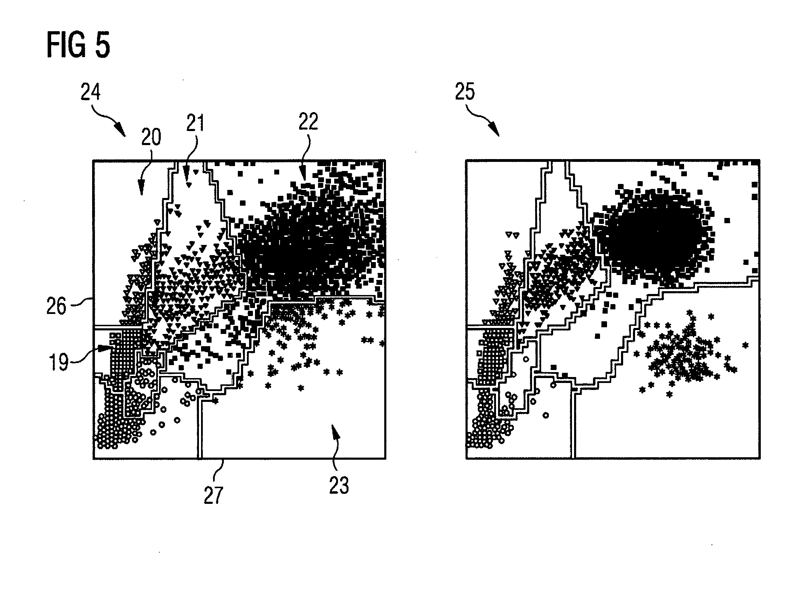

Figur 5 die Ergebnisse einer Analyse nach Peroxidase-Einfärbung mit dem Gerät ADVIA 2120i, das beispielhaft die Volumen- und Dichteverteilung (PEROX Y 26 bzw. PEROX X 27) zwischen einer Malaria-infizierten Probe 24 und einer normalen Probe 25 darstellt. "PEROX Y" 26 bezeichnet die Breite der Peroxidase-negativen Populationen entlang der Y-Achse. Diese erscheint bei einer Malariaprobe länger im Vergleich zu einer normalen Probe. Die Ergebnisse für die Malariaprobe 24 wirken diffuser als die normale Probe 25, was auf die größere Volumenverteilung 26 und Dichteverteilung 27 zurückzuführen ist.

Im Folgenden wird beispielhaft die Durchführung des erfindungsgemäßen Verfahrens dargestellt, sowie zwei Algorithmen, die auf Grundlage des erfindungsgemäßen Verfahrens die Bewertung der ermittelten Parameter und eine Vorhersage einer Plasmodieninfektion mit hoher Spezifität und Sensitivität ermöglichen. Die Messungen wurden mit dem ADVIA 2120i-System der SIEMENS AG durchgeführt. Die folgenden Abkürzungen werden verwendet:

| **Parameter** | **Beschreibung** |
|---|---|
| PMNx | Dichteverteilung der polymorphkernigen Leukozyten |
| MPC | Verteilung der Zelldichte der Thrombozyten |
| PLT | Gesamtmenge der Thrombozyten in der Blutprobe |
| PLT mode | Geräteeinstellung von ADVIA 2120i |
| %Baso Noise | Unspezifischer Anteil aus dem Streulichtdiagramm (Klein- und Großwinkelstreuung) nach spezifischer Lyse durch BASO Lysereagenz |
| %Abnorm | Standardabweichung der Clusterverteilung alle Leukozyten |
| PEROX Y Sigma | Volumenverteilung der neutrophilen Granulozyten nach Peroxidase-Einfärbung |

Gemäß einer ersten erfindungsgemäßen Auswertung ergab sich folgender Algorithmus 1 für ein Nachweisverfahren für eine Malariainfektion mit hoher Spezifität:
PLT x MPC / 100 < 47 und
PMNx + 0,1635 x (PLT x MPC / 100)< 33

Anhand dieses Algorithmus zeigt sich deutlich (siehe unten), dass das zueinander ins Verhältnis setzen der Ergebnisse einer Differentialanalyse der polymorphkernigen neutrophilen Granulozyten bezüglich der Verteilung der Zelldichte (PMNx) mit der Thrombozytenanzahl und der Verteilung der Zelldichte der Thrombozyten in der Probe gegebenenfalls unter Hinzuziehung weiterer Unteralgorithmen (1b und 1c) zu einer sehr hohen Spezifität führt.

Unter Hinzuziehung der weiteren Unteralgorithmen
PLT mode - 30 x %Baso Noise < 27 sowie
Perox Y Sigma + 12/70 x %Abnorm > 12
ergab sich eine Testspezifität für Malaria von 99 % sowie eine Sensitivität von 76,5 %.

Algorithmus 1 kann insofern in vorteilhafter in Ländern mit einer hohen Prävalenz von Malaria verwendet werden.

Für Länder mit einer niedrigen Malariaprävalenz würde sich der folgende Algorithmus 2 anbieten:
2a. PMN peak + 14 / 140 x (PLT x MPC)/100 < 34
2b. Baso Noise >0,074
2c. PLT Mode + 25 x %Baso Noise < 45
2d. Perox Y Sigma > 6,6

Die beobachtete Spezifität betrug 90,2 %, die Sensitivität 98,9 %. Mit anderen Worten kann durch den Algorithmus 2 eine extrem hohe Sensitivität erreicht werden.

Damit können durch den Algorithmus 1 in einer großen Probenpopulation beinahe alle Malaria positiven Proben erkannt werden, wohingegen sich Algorithmus 2 insbesondere für Screening-Untersuchungen eignet. Auch eine Kombination beider Algorithmen kann zusätzliche Vorteile mit sich bringen.

### Bezugszeichenliste

- 1: Vorrichtung zur Durchflusszytometrie
- 2: Laser
- 3: Sensormodul
- 4: Optische Linsen
- 5: halbduchlässiger Spiegel
- 6: Blende
- 7: Spiegel
- 8: Sensor
- 9: Sensor
- 10: Sensor
- 11: Zelle
- 12: Baso Volumen
- 13: Baso Konfiguration
- 14: Großwinkel PLT Scatter
- 15: Kleinwinkel PLT Scatter
- 16: Thrombozyten Volumen
- 17: Thrombozyten Komponenten
- 18: Unspezifischer Anteil aus dem Streulichtdiagramm
- 19: Lymphozyten
- 20: Große ungefärbte Zellen
- 21: Monozyten
- 22: Neutrophile Leukozyten
- 23: Eosinophile Leukozyten
- 24: Malariaprobe
- 25: Normale Probe
- 26: Peroxy-Sigma
- 27: Absorption

## Patentansprüche

1. Verfahren zum Nachweis einer Plasmodieninfektion in einer Patientenblutprobe, umfassend die Schritte:
a) Durchführen einer Differentialanalyse der polymorphkernigen neutrophilen Granulozyten in der Probe und Bestimmen der Verteilung des Zellvolumens und der Zelldichte;
b) Bestimmen der Thrombozytenanzahl in der Probe;
c) Bestimmen der Verteilung der Zelldichte der Thrombozyten in der Probe;
d) Gewinnen von Probenparametern aus den in a) - c) durchgeführten Bestimmungen; und
e) Bewerten der Parameter gegenüber einem vorherbestimmten Kriterium, wobei bei Erfüllung des Kriteriums eine Plasmodieninfektion vorliegt, wobei das Kriterium auf Grundlage einer oder mehrerer Probenparameter etabliert wird und auf Grundlage eines Vergleichs von infizierten Blutproben mit entsprechenden Werten von normalen Blutproben bestimmt wird.

2. Verfahren nach Anspruch 1, wobei die Bestimmung der Parameter durch Streulichtmessung erfolgt.

3. Verfahren nach Anspruch 1 oder 2, wobei es sich bei der Plasmodieninfektion um eine Infektion mit Plasmodium falciparum, Plasmodium vivax, Plasmodium ovale, Plasmodium malariae oder Plasmodium knowlesi handelt.

4. Verfahren nach einem oder mehreren der vorhergehenden Ansprüche, wobei zusätzlich eine Sphärisierung und Differentialanalyse der Retikulozyten und Erythrozyten durch Streulichtmessung und Absorptionsmessung erfolgt.

5. Verfahren nach einem oder mehreren der vorhergehenden Ansprüche, wobei zusätzlich nach einer -Einfärbung aller Leukozyten in der Probe eine Differentialanalyse durch Streulichtmessung und Absorption erfolgt.

6. Verfahren nach Anspruch 5, wobei die Einfärbung mittels 4-Chlor-1-naphthol als Substrat durchgeführt wird.

7. Verfahren nach einem oder mehreren der vorhergehenden Ansprüche, wobei zusätzlich nach einer -Einfärbung die Bestimmung der Volumenverteilung der neutrophilen Granulozyten erfolgt.

8. Verfahren nach einem oder mehreren der vorhergehenden Ansprüche, wobei zusätzlich als Parameter die Standardabweichung der Volumenverteilung und Absorptionsverteilung aller Leukozyten nach Einfärbung verwendet wird.

9. Verfahren nach einem oder mehreren der vorhergehenden Ansprüche, wobei die Bestimmung in a) nach Lysieren wenigstens der eosinophilen und neutrophilen, nicht jedoch der basophilen Leukozyten, durch ein geeignetes Lysereagenz erfolgt.

10. Verfahren nach einem oder mehreren der vorhergehenden Ansprüche, wobei nach einer spezifischen Lyse aller Zellen außer der basophilen Leukozyten der unspezifische Anteil aus dem Streulichtdiagramm (Klein- und Großwinkelstreuung) bestimmt wird.

11. Verfahren nach einem oder mehreren der vorhergehenden Ansprüche, wobei niedrige Werte der in a), b) und c) bestimmten Parameter prädikativ für das Vorliegen einer Plasmodieninfektion sind.

12. Verfahren nach Anspruch 7,8 und 10, wobei eine Erhöhung der Parameter prädiktiv für das Vorliegen einer Plasmodieninfektion ist.

13. Verfahren nach einem oder mehreren der vorhergehenden Ansprüche, das mit einem automatisierten Zellzählgerät durchgeführt wird.

## Claims

1. Method of detecting a *Plasmodium* infection in a patient's blood sample, comprising the steps of:
a) performing a differential analysis of polymorphonuclear neutrophil granulocytes in the sample and determining the distribution of cell volume and cell density;
b) determining the number of platelets in the sample;
c) determining the distribution of platelet cell density in the sample;
d) obtaining sample parameters from the determinations carried out in a) - c); and
e) evaluating said parameters with respect to a predetermined criterion, with a *Plasmodium* infection being present when the criterion is fulfilled, said criterion being established on the basis of one or more sample parameters and being determined on the basis of a comparison of infected blood samples with corresponding values from normal blood samples.

2. Method according to Claim 1, wherein the parameters are determined by measuring scattered light.

3. Method according to Claim 1 or 2, wherein the *Plasmodium* infection is an infection by *Plasmodium falciparum, Plasmodium vivax, Plasmodium ovale, Plasmodium malariae* or *Plasmodium knowlesi.*

4. Method according to one or more of the preceding claims, wherein additionally spherization and differential analysis of reticulocytes and erythrocytes by measuring scattered light and absorption are carried out.

5. Method according to one or more of the preceding claims, wherein additionally a differential analysis by measuring scattered light and absorption is carried out after staining of all leukocytes in the sample.

6. Method according to Claim 5, wherein staining is performed by means of 4-chloro-1-naphthol as substrate.

7. Method according to one or more of the preceding claims, wherein additionally the volume distribution of neutrophil granulocytes is determined after staining.

8. Method according to one or more of the preceding claims, wherein additionally the standard deviation of volume distribution and absorption distribution of all leukocytes after staining is used as parameter.

9. Method according to one or more of the preceding claims, wherein the determination in a) is carried out after lysis by a suitable lysis reagent of at least the eosinophil and neutrophil but not the basophil leukocytes.

10. Method according to one or more of the preceding claims, wherein, after a specific lysis of all cells except basophil leukocytes, the unspecific portion is determined from the scattered-light plot (small-angle and wide-angle scattering).

11. Method according to one or more of the preceding claims, wherein low values of the parameters determined in a), b) and c) are predictive of the presence of a *Plasmodium* infection.

12. Method according to Claims 7, 8 and 10, wherein an increase in the parameters is predictive of the presence of a *Plasmodium* infection.

13. Method according to one or more of the preceding claims, which is performed using an automated cell counter.

## Revendications

1. Procédé de détection d'une infection plasmodiale dans un échantillon de sang d'un patient, comprenant les stades dans desquels :
a) on effectue une analyse différentielle des granulocytes neutrophiles à noyau polymorphe dans l'échantillon et on détermine la répartition du volume de cellule et de la densité de cellule ;
b) on détermine le nombre de thrombocytes dans l'échantillon ;
c) on détermine la répartition de la densité de cellule des thrombocytes dans l'échantillon ;
d) on obtient des paramètres d'échantillon à partir des déterminations effectuées dans a) à c) ; et
e) on estime les paramètres par rapport à un critère déterminé à l'avance, dans lequel, si le critère est satisfait, il y a une infection plasmodiale, le critère étant établi sur la base d'un ou de plusieurs paramètres d'échantillon et étant déterminé sur la base d'une comparaison d'échantillons de sang infecté à des valeurs correspondantes d'échantillons de sang normal.

2. Procédé suivant la revendication 1, dans lequel on effectue la détermination des paramètres par une mesure en lumière diffusée.

3. Procédé suivant la revendication 1 ou 2, dans lequel l'infection plasmodiale est une infection par plasmodium falciparum, plasmodium vivax, plasmodium ovalé, plasmodium malariae ou plasmodium knowlesi.

4. Procédé suivant l'une ou plusieurs des revendications précédentes, dans lequel on effectue, en outre, une sphérisation et une analyse différentielle des réticulocytes et des érythrocytes par une mesure par diffusion de lumière et par une mesure par absorption.

5. Procédé suivant l'une ou plusieurs des revendications précédentes, dans lequel on effectue, en outre, après une coloration de tous les leucocytes dans l'échantillon, une analyse différentielle par mesure par diffusion de lumière et par absorption.

6. Procédé suivant la revendication 5, dans lequel on effectue la coloration au moyen du 4-chloro-1-naphtol comme substrat.

7. Procédé suivant l'une ou plusieurs des revendications précédentes, dans lequel on effectue, en outre, après une coloration, la détermination de la répartition en volume des granulocytes neutrophiles.

8. Procédé suivant l'une ou plusieurs des revendications précédentes, dans lequel on utilise, en outre, comme paramètre l'écart-type de la répartition en volume et de la répartition en absorption de tous les leucocytes après la coloration.

9. Procédé suivant l'une ou plusieurs des revendications précédentes, dans lequel on effectue la détermination dans a) après avoir lysé au moins les leucocytes éosinophiles et neutrophiles, mais non les leucocytes basophiles, par un réactif de lyse approprié.

10. Procédé suivant l'une ou plusieurs des revendications précédentes, dans lequel, après une lyse spécifique de toutes les cellules, à l'exception des leucocytes basophiles, on détermine la proportion non spécifique à partir du diagramme de diffusion de la lumière (diffusion sous un petit angle et diffusion sous un grand angle).

11. Procédé suivant l'une ou plusieurs des revendications précédentes, dans lequel des valeurs basses des paramètres déterminés dans a), b) et c) prédisent la présence d'une infection plasmodiale.

12. Procédé suivant les revendications 7, 8 et 10, dans lequel une augmentation des paramètres prédit la présence d'une infection plasmodiale.

13. Procédé suivant l'une ou plusieurs des revendications précédentes, qui est effectué par un appareil automatisé de comptage des cellules.
